# FASCICULE DE BREVET EUROPEEN

(11) **EP 0 504 347 B1**
(45) Date de publication et mention de la délivrance du brevet: **28.12.1994**
(21) Numéro de dépôt: 91916995.3
(22) Date de dépôt: 19.09.1991
(51) Int. Cl.: A61K 7/00

(54) **COMPOSITION COSMETIQUE COMPORTANT UNE DISPERSION DE VESICULES LIPIDIQUES AINSI QUE DES PIGMENTS MELANIQUES**
EINE LIPIDVESIKELDISPERSION UND MELANINPIGMENTE ENTHALTENDES KOSMETIKUM
COSMETIC COMPOSITION COMPRISING A DISPERSION OF LIPIDIC VESICLES AS WELL AS MELANIC PIGMENTS

(30) Priorité: 27.09.1990 LU 87814
(43) Date de publication de la demande: 23.09.1992
(73) Titulaire: L'OREAL, 75008 Paris (FR)
(72) Inventeur: GROLLIER, Jean-François, F-75004 Paris (FR)
(74) Mandataire: Peuscet, Jacques
(86) Numéro de dépôt international: FR9100738
(87) Numéro de publication internationale: WO9205761

(56) Documents cités:
- EP-A- 0 313 380
- EP-A- 0 379 409
- EP-A- 0 386 680
- GB-A- 2 207 153

## Description

### UTILISATION.

La présente invention est relative à une composition cosmétique à base de vésicules lipidiques, ladite composition renfermant des pigments mélaniques, ainsi qu'à son utilisation, notamment pour la protection des matières kératiniques, en particulier de la peau et des cheveux, contre les radiations ultraviolettes, et pour la pigmentation de la peau et des cheveux afin notamment de les colorer d'une couleur proche de celle de la pigmentation naturelle.

Des compositions à base de vésicules lipidiques sont déjà connues, notamment dans le domaine précité. Les vésicules lipidiques, se présentant généralement sous la forme de dispersions aqueuses, sont caractérisées par une structure lamellaire de feuillets constitués d'au moins une bi-couche lipidique, et encapsulant une phase aqueuse pouvant comprendre des substances actives hydrosolubles, lesquelles sont ainsi protégées des conditions extérieures.

Les vésicules peuvent être obtenues à partir de lipides ioniques et/ou de lipides non-ioniques : le terme "vésicule" utilisé dans la présente demande englobe tous les cas.

La protection naturelle de la peau humaine contre les radiations ultraviolettes est fonction de la quantité de pigment mélanique présent dans les mélanocytes, et ce taux varie suivant les différents types de peau, claires à foncées. Ce rôle photoprotecteur de la mélanine est bien connu. C'est ainsi que chez les individus présentant une peau de type I, II, III, qui ne produit pas la mélanine en quantité suffisante, l'exposition prolongée au soleil peut entraîner un vieillissement prématuré de la peau et des cancers cutanés.

Pour pallier l'insuffisance de ce pigment dans l'épiderme, on a déjà proposé d'appliquer de la mélanine sur la peau. Cependant, l'application topique de la mélanine pose des problèmes au niveau de sa mise en oeuvre dans les compositions, en raison d'une mise en dispersion et d'une compatibilité difficiles dans les supports utilisés en cosmétique. La complexité de ces problèmes limite donc son utilisation.

Par ailleurs, toujours pour pallier l'insuffisance de ce pigment dans l'épiderme, on a également proposé, conformément aux demandes de brevets français FR-A-2 623 716 et FR-A-2 624 374, d'utiliser dans les compositions bronzantes ou solaires, des précurseurs de mélanine, tels que la L-tyrosine ou des dérivés hydrosolubles de la L-tyrosine, qui, en franchissant la barrière cutanée, sont transformés en L-tyrosine métabolisable et conduisent à la formation de mélanine dans la peau humaine. Les demandes de brevet précitées proposent ainsi des compositions à base de phases lamellaires lipidiques hydratées ou de liposomes contenant de la tyrosine ou un dérivé de tyrosine. L'efficacité de telles compositions est néanmoins limitée en raison de l'utilisation d'un précurseur de pigment mélanique. Dans EP-A 0 386 680 est décrit un procédé de bronzage de la peau qui consiste à appliquer sur la peau une composition contenant une certaine quantité de mélanine complexée avec des liposomes, la mélanine étant soit dans les parois lipidiques des liposomes, soit dans la phase aqueuse encapsulée.

Selon l'invention, on a maintenant découvert d'une façon surprenante qu'une composition sous forme de dispersion de vésicules de lipides amphiphiles ioniques et/ou non-ioniques, contenant dans la phase externe au moins un pigment mélanique déjà formé, permettait de remédier aux problèmes rencontrés jusqu'ici en constituant une composition cosmétique avantageuse sous plusieurs aspects :
- elle permet l'obtention d'une dispersion très homogène du pigment mélanique qui peut ainsi être réparti uniformément sur la peau ou les cheveux ;
- elle permet l'obtention d'une composition stable notamment sous irradiation U.V. ; on a en particulier observé qu'il n'y a pas de peroxydation donc pas de rancissement des corps gras ;
- elle augmente la durée de la protection des matières kératiniques contre les effets nocifs des radiations ultraviolettes et permet de potentialiser l'effet protecteur ;
- elle favorise le bronzage de la peau et lui communique une couleur uniforme et un meilleur aspect ;
- elle colore les cheveux gris ; et
- elle engendre un stockage amélioré du pigment mélanique dans la couche cornée.

La présente invention a donc pour objet une composition cosmétique comportant, en dispersion aqueuse, des vésicules lipidiques à structure lamellaire encapsulant une phase aqueuse, caractérisée par le fait qu'elle contient au moins un pigment mélanique, ayant une dimension de particule comprise entre environ 500 nm et 50.000 nm, en mélange avec les vésicules dans la phase externe de la dispersion.
Une partie de ce pigment mélanique peut éventuellement être présent dans la phase encapsulée des vésicules.

Le (ou les) pigment(s) mélanique(s) peuvent être obtenus (A) par oxydation d'au moins un composé indolique, (B) par polymérisation oxydante ou enzymatique de précurseurs mélaniques, ou (C) par extraction de la mélanine à partir de substances en contenant.
(A) Les pigments mélaniques peuvent, en premier lieu, être obtenus par oxydation d'au moins un composé indolique choisi notamment parmi ceux répondant à la formule (I) : 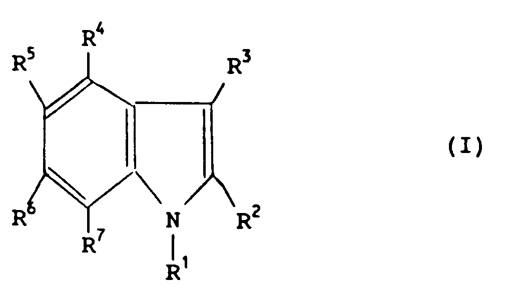 dans laquelle :
   - R¹ et R³ représentent, indépendamment l'un de l'autre, hydrogène ou alkyle en C₁-C₄ ;
   - R² représente hydrogène, alkyle en C₁-C₄, carboxyle ou (alcoxy en C₁-C₄)-carbonyle ;
   - R⁴ et R⁷ représentent, indépendamment l'un de l'autre, hydrogène, hydroxy, alkyle en C₁-C₄, amino, alcoxy en C₁-C₄, (acyl en C₂-C₄)-oxy, (acyl en C₂-C₄)-amino ;
   - R⁵ représente hydrogène, hydroxy, alcoxy en C₁-C₄, alkyle en C₁-C₄, halogène, amino, (acyl en C₂-C₁₄)-oxy, (acyl en C₂-C₄)-amino, triméthylsilyloxy ;
   - R⁶ représente hydrogène, hydroxy, alcoxy en C₁-C₄, amino, (acyl en C₂-C₄)-oxy, (acyl en C₂-₄)-amino, triméthylsilyloxy, (hydroxyalkyl en C₂-C₄)-amino ;
   - R⁵ et R⁶ pouvant également former, conjointement avec les atomes de carbone auxquels ils sont rattachés, un cycle méthylènedioxy éventuellement substitué par un groupement alkyle en C₁-C₄ ou alcoxy en C₁-C₄, ou bien un cycle carbonyldioxy :
   - au moins l'un des radicaux R⁴ à R⁷ représente un groupement OZ ou NHR⁰ avec au plus un des radicaux R⁴ à R⁷ représentant NHR⁰ ; et au plus deux des radicaux R⁴ à R⁷ représentent OZ et, dans le cas où Z représente hydrogène, les deux OH sont dans les positions 5 et 6 ; et au moins l'un des radicaux R⁴ à R⁷ représente hydrogène, et dans le cas ou un seul de ces radicaux représente hydrogène, un seul radical parmi R⁴ à R⁷ représente alors NHR⁰ ou OZ, les autres radicaux représentant alkyle en C₁-C₄ ;
   - le R⁰ du groupement NHR⁰ désignant hydrogène, acyle en C₂-C₄, hydroxyalkyle en C₂-C₄, et le Z du groupement OZ désignant hydrogène, acyle en C₂-C₁₄, alkyle en C₁-C₄, ou triméthylsilyle,
   et leurs sels de métaux alcalins, alcalino-terreux, d'ammonium ou d'amines.
   Ces composés indoliques sont choisis, de préférence, parmi le 4-hydroxyindole, le 5-hydroxyindole, le 6-hydroxyindole, le 7-hydroxyindole, le 4-hydroxy 5-méthoxyindole, le 4-hydroxy 5-éthoxyindole, le 2-carboxyl 5-hydroxyindole, le 5-hydroxy 6-méthoxyindole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxyl 5,6-dihydroxyindole, le 4-hydroxy 5-méthyl indole, le 2-carboxyl 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxyl 5-méthylindole, le 6-hydroxy 2-éthoxycarbonyl 5-méthoxyindole, le 6-N-β-hydroxyéthylaminoindole, le 4-aminoindole, le 5-aminoindole, le 6-aminoindole, le 7-aminoindole, le N-méthyl 6β-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6-diacétoxyindole, le 5-méthoxy 6-acétoxyindole, le 5,6-diméthoxyindole, le 5,6-méthylènedioxyindole, le 5,6-triméthylsilyloxyindole, l'ester phosphorique du 5,6-dihydroxyindole et le 5,6 dibenzyloxyindole, et les sels d'addition de ces composés.
   Le 5,6-dihydroxyindole et le 6-hydroxyindole sont particulièrement préférés.
   L'oxydation du composé indolique de formule (I) peut s'effectuer en milieu aqueux ou eau-solvant(s), à l'air, en présence ou non d'un agent alcalin et/ou d'un catalyseur métallique d'oxydation tel, par exemple l'ion cuivrique.
   Le milieu réactionnel est, de préférence, constitué par de l'eau et peut, le cas échéant, être constitué par un mélange d'eau et d'au moins un solvant choisi de telle façon qu'il solubilisera rapidement le composé indolique de formule (I). Parmi ces solvants, on peut citer, à titre d'exemples, les alcools inférieurs en C₁-C₄, tels que l'alcool éthylique, l'alcool propylique ou isopropylique, l'alcool tert.-butylique, les alkylèneglycols, tels que l'éthylèneglycol, le propylèneglycol, les alkyléthers d'alxylèneglycols, tels que les éthers monométhylique, monoéthylique et monobutylique de l'éthylèneglycol, les monométhyléthers du propylèneglycol et du dipropylèneglycol, et le lactate de méthyle.
   L'oxydation peut également s'effectuer en mettant en oeuvre le peroxyde d'hydrogène en présence d'un agent alcalin, tel que, de préférence, l'ammoniaque, ou en présence d'un ion iodure, l'iodure étant, de préférence, l'iodure d'un métal alcalin, alcalino-terreux ou d'ammonium.
   On peut également procéder à l'oxydation en utilisant l'acide periodique et ses sels hydrosolubles et dérivés, les permanganates et bichromates, tels que de sodium ou de potassium, l'hypochlorite de sodium, le ferricyanure de potassium, le persulfate d'ammonium, l'oxyde d'argent, l'oxyde de plomb, le chlorure ferrique, le nitrite de sodium, les sels de terres rares dont, notamment, le cérium, et des oxydants organiques choisis parmi les ortho- et parabenzoquinones, les ortho- et parabenzoquinones mono- ou diimines, les 1,2- et 1,4-naphtoquinones, les 1,2- et 1,4-naphtoquinones mono- ou diimines telles que définies dans la demande EP-A-0 376 776. Le sel d'acide periodique préféré est le periodate de sodium.
   Il est possible d'activer les agents oxydants par un modificateur de pH.
   On peut également envisager une oxydation enzymatique.
   Le produit insoluble est isolé par filtration, centrifugation, lyophilisation ou atomisation ; il est ensuite broyé ou micronisé pour atteindre la granulométrie désirée.
(B) Les pigments mélaniques selon l'invention peuvent également provenir de la polymérisation oxydante ou enzymatique de précurseurs mélaniques, tels que la L-tyrosine, la L-dopa, le catéchol et leurs dérivés.
(C) Les pigments mélaniques selon l'invention peuvent enfin provenir de l'extraction de la mélanine de substances telles que les cheveux humains, l'encre de céphalopodes (seiches, poulpes) encore connue sous le nom de sépiomélanine, auquel cas le pigment est broyé et purifié avant son utilisation.

On utilise, de préférence, les pigments cités dans le paragraphe (A) obtenus par oxydation d'au moins un composé indolique et dans le paragraphe (C) obtenus par extraction de la mélanine à partir de substances en contenant.

Conformément à une mise en oeuvre particulière de l'invention, le (ou les) pigment(s) mélanique(s) est (ou sont) associé(s) à au moins une charge particulaire pour constituer un pigment mélanique composite. Dans ce cas, le (ou les) pigment(s) mélanique(s) peut (ou peuvent) résulter de l'oxydation d'au moins un composé indolique de formule (I), telle que définie ci-dessus, en mélange avec la charge, dans un milieu essentiellement non-solvant de ladite charge, à une température pouvant aller de la température ambiante jusqu'à environ 100°C, ou encore de la polymérisation oxydante de précurseurs mélaniques sur la charge.

Les conditions générales de l'oxydation des composés indoliques de formule (I) sont les mêmes que celles mentionnées ci-dessus.

Selon un premier mode de réalisation, la charge particulaire est une charge minérale inerte de granulométrie inférieure à 20 000 nm, notamment inférieure à 10 000 nm et voisine de 5 000 nm, constituée avantageusement de particules de carbonate de calcium, de silice ou d'oxyde de titane. De tels pigments mélaniques composites, déposés sur charge minérale, sont décrits, ainsi que leur préparation, dans la demande de brevet français FR-A-2 618 069.

Selon un deuxième mode de réalisation de la présente invention, la charge particulaire est une charge polymérique inerte, de granulométrie inférieure à 100 000 nm, notamment de 10 à 50 000 nm, choisie avantageusement parmi les polymères naturels ou synthétiques, organiques ou inorganiques, à réseau réticulé, cristallin ou amorphe, ayant un poids moléculaire compris entre 5000 et 5 000 000. Des pigments mélaniques composites sur charge polymérique ainsi que leur préparation sont décrits dans la demande de brevet luxembourgeois LU-A- 87 429.

Les polyméres organiques ou synthétiques sont, en particulier, choisis parmi les polymères dérivés de la kératine, de la chitine ou de la cellulose, ou parmi les polyamides ou des homo- ou copolymères résultant de la polymérisation de monomères mono- ou polyéthyléniques, aliphatiques ou aromatiques, à réseau réticulé, cristallin ou amorphe.

Les polymères dérivés de la kératine sont choisis parmi les kératines animales ou humaines. D'autres polymères dérivés de la kératine utilisables sont des kératines modifiées chimiquement, ayant un poids moléculaire compris entre 10 000 et 250 000 et, en particulier, la kératine partiellement hydrolysée (ou hydrolysat de kératine), ayant un poids moléculaire compris entre 50 000 et 200 000 ; cet hydrolysat est, de préférence, obtenu par hydrolyse alcaline modérée ; des produits de ce type sont, par exemple, vendus sous la dénomination de "KERASOL" par la Société "CRODA". D'autres kératines modifiées sont les kératines sulfoniques d'un poids moléculaire compris entre 10 000 et 100 000, obtenues par oxydation de tout ou partie des liaisons disulfure des groupements cystine de la kératine en groupements acide cystéique. On peut également mentionner, comme dérivé kératinique, la fibroîne de soie.

Les polymères dérivés de la chitine comprennent d'abord la chitine, qui est un polymère naturel, et le dérivé désacétylé de la chitine connue sous la dénomination de chitosane, obtenu par saponification des groupements acétyle de la chitine. Le chitosane, tel que proposé dans le commerce, est partiellement acétylé et contient 70 à 90 % en poids de chitosane. On peut également l'utiliser sous la forme de sels insolubles, tels que les sulfates et phosphates. Des produits de ce type sont vendus, par exemple, sous la dénomination de "KYTEX" par la Société "HERCULES".

Les polymères cellulosiques sont choisis plus particulièrement parmi les celluloses microcristallines, telles que les produits vendus sous la dénomination d'"AVICEL" par la société "FMC CORPORATION".

Parmi les polymères synthétiques, on peut tout particulièrement citer le polyéthylène, le polypropylène, le polystyrène, le poly(méthacrylate de méthyle) vendus sous les dénominations "MICROPEARL M" et "MICROPEARL M100" par la Société "SEPPIC", le poly(méthacrylate de méthyle) réticulé, tel que le produit vendu sous la dénomination de "MICROPEARL M 305" par la Société "SEPPIC". D'autres polymères sont, en particulier, choisis parmi la poly-β-alanine réticulée, telle que décrite dans le brevet français 2 530 250, ou encore présentée avantageusement sous forme de microsphères présentant une très faible dispersité de taille, 85 % en poids ayant une granulométrie comprise entre 28.000 et 46.000 nm, et dont le taux de réticulation est compris entre 1 et 15 % et de préférence, entre 1 et 8 %.

On peut également utiliser, à titre de polymères, des produits connus sous la dénomination de microéponges, tels que des polymères réticulés de styrène/divinylbenzène ou de méthacrylate de méthyle/diméthacrylate d'éthylène-glycol ou de stéarate de vinyle/divinylbenzène, tels que décrits dans les brevets WO-88/01164 et US-A-4 690 825. De tels polymères sont essentiellement constitués par des billes de polymères réticulés comprenant un réseau interne de pores, capable de retenir le pigment mélanique. D'autres polymères de ce type sont des microsphères creuses d'un copolymère de chlorure de vinylidène et d'acrylonitrile, vendues sous la dénomination d' "EXPANCEL" par la Société "KEMA NORD" ; ou encore des microsphères poreuses de polyamide 12, de polyamide 6 ou de copolyamide 6/12, vendues sous la dénomination d' "ORGASOL" par la Société "ATOCHEM" ; ces microsphères ont, de préférence, une granulométrie comprise entre 10 000 et 50 000 nm.

On peut utiliser également des poudres de silicone qui sont des gommes, des résines et, plus particulièrement, des élastomères d'organopolysiloxane.

Selon un troisième mode de réalisation, la charge particulaire est une charge constituée par des particules à structure lamellaire, organiques ou minérales, ayant une dimension inférieure à 50 000 nm.

Les particules de structure lamellaire, constituées de feuillets pour lesquels le rapport entre la plus grande dimension et l'épaisseur est notamment compris entre 2 et 100, sont choisies en particulier parmi les produits suivants : la L-lauroyllysine, telle que le produit vendu sous la dénomination d'"AMIHOPE L.L." par la Société "AJINOMOTO" : les microparticules de céramique éventuellement recouvertes de poudre de zirconium, telles que les produits vendus sous les appellations de "TORAYCERAM ZP 550" et "ZP 4000" par la Société "TORAY" ; le dioxyde de titane lamellaire, tel que les produits vendus sous les dénominations de "LUXELEN SILK D" et "LUXELEN SS" par la Société "SUMITOMO", le talc lamellaire, le nitrure de bore, tel que les produits vendus respectivement sous les dénominations "Nitrure de bore SF" ou "SHP" par les Sociétés "WACKER" et "KAWASAKI" : le mica lamellaire, tel que le produit vendu sous la dénomination de "MICA CONCORD 1000" par la Société "SCIAMA" ; l'oxychlorure de bismuth, tel que le produit vendu sous la dénomination de "PEARL GLO" par la Société "MALLINCKRODT". La dimension des particules de structure lamellaire utilisées conformément à l'invention, est, de préférence, inférieure à 50 000 nm et, en particulier, inférieure à 25 000 nm ; leur dimension est généralement supérieure à 500 nm ; elle est comprise, en particulier, entre 1 000 et 20 000 nm. Ces particules ont une épaisseur généralement supérieure à 10 nm. Ces particules lamellaires peuvent se présenter sous forme d'une structure stratifiée. Des détails et compléments concernant la structure et la préparation de ces pigments mélaniques sur charges lamellaires sont décrits dans la demande de brevet FR 90-090 053 déposée le 16 juillet 1990 au nom de la Société déposante.

Les vésicules des compositions conformes à l'invention sont préparées à partir d'une phase lipidique comprenant au moins un lipide amphiphile ionique et/ou un lipide amphiphile non-ionique associé éventuellement à au fins un additif stabilisant, lesdites vésicules contenant une phase aqueuse encapsulée, laquelle peut contenir des produits cosmétiquement actifs tels que des agents hydratants ou des agents apaisants.

Parmi les lipides amphiphiles non-ioniques utilisables, on peut citer
(1) les éthers de polyglycérol, linéaires ou ramifiés, de formule : 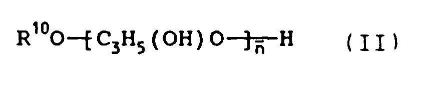 dans laquelle :
   · -C₃H₅(OH)O est représenté par les structures suivantes prises en mélange ou séparément : -CH₂CHOHCH₂O- ; 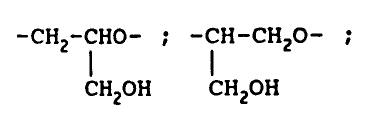
   · n̅ est une valeur statistique moyenne comprise entre 2 et 6 ;
   · R¹⁰ représente :
      (a) une chaîne aliphatique, linéaire ou ramifiée, contenant de 12 à 18 atomes de carbone ;
      (b) un reste R¹¹CO, où R¹¹ est un radical aliphatique, linéaire ou ramifié, en C₁₁-C₁₇ ;
      (c) un reste R¹²-[-OC₂H₃(R¹³)-]-, où :
         · R¹² peut prendre la signification (a) ou (b) donnée pour R¹⁰ ;
         · OC₂H₃(R¹³)- est représenté par les structures suivantes, prises en mélange ou séparément : 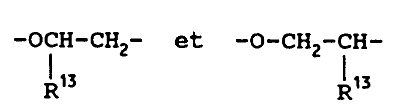 où R¹³ prend la signification (a) donnée pour R¹⁰ ;
(2) les alcools gras polyoxyéthylénés, les stérols polyoxyéthylénés ;
(3) les esters de polyols éventuellement polyoxyéthylénés ;
(4) les cérébrosides ; et
(5) le stéarate de polyglycérol oxyéthyléné.

Parmi les lipides amphiphiles ioniques utilisables, on peut citer :
- les phosphoaminolipides ;
- les glycolipides ;
- les phospholipides naturels, tels que la lécithine d'oeuf ou de soja, la sphingomyéline, la phosphatidylsérine, la dipalmitoylphosphatidylcholine et les lécithines hydrogénées.

On utilise, de préférence, les lipides amphiphiles non-ioniques ci-dessus et, en particulier, les éthers de polyglycérol de formule (II).

L'additif stabilisant est destiné, de façon connue, à modifier la perméabilité et/ou la charge superficielle des vésicules. Il est, de préférence, choisi dans le groupe formé par les stérols et les stabilisants anioniques. Le stérol est avantageusement le cholestérol. Le stabilisant anionique est avantageusement choisi, d'une part, parmi les sels monosodiques ou disodiques d'(acyl en C₁₄-C₂₂)-glutamates, tel que le sel monosodique de l'acide N-stéaroylglutamique, les sels disodiques avec des radicaux acyles du coprah et du suif ou encore les radicaux cocoyle et stéaroyle, et, d'autre part, parmi les esters phosphoriques d'alcools gras en C₁₂-C₂₂. De façon connue, on peut ajouter au(x) lipide(s) amphipile(s) à la fois un stérol et un stabilisant anionique.

Les vésicules ont avantageusement un diamètre moyen compris entre 10 et 1000 nm.

La phase lipidique des vésicules, constituée par les lipides et, le cas échéant le (ou les) stabilisant(s) associé(s) à ceux-ci, représente avantageusement de 0,1 à 16% et, de préférence, de 0,1 à 10% du poids total de la composition ; le(s) lipide(s) amphiphile(s) ionique(s) et/ou non-ionique(s) représente(nt) avantageusement de 0,1 à 16% et, de préférence, de 0,1 à 10% du poids total de la composition ; et l'additif stabilisant représente avantageusement moins de 10% du poids total de la composition.

Selon l'invention, le pigment mélanique est introduit, sous agitation, dans la dispersion de vésicules déjà formées.

Dans les compositions selon l'invention, le (ou les) pigment(s) mélanique(s), éventuellement sur charge, est (ou sont) présent(s) dans des proportions comprises notamment entre 0,01 et 5 % en poids par rapport au poids total de la composition.

Les vésicules sont présentes dans des proportions comprises entre 0,5 et 15 % en poids par rapport au poids total de la composition.

La composition selon l'invention peut se présenter sous forme de dispersion plus ou moins épaisse, de gel, de crème et de lait.

La composition selon l'invention peut également renfermer, outre le (ou les) pigment(s) mélanique(s) et les vésicules, des additifs cosmétiquement acceptables tels que la dihydroxyacétone, des précurseurs de mélanine tels que la tyrosine ; des silicones, notamment des gommes de silicone et des mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA), tel que le produit vendu sous la dénomination de "Q2-1401" par la Société "DOW CORNING" ; des agents épaississants, tels que les acides polyacryliques réticulés notamment ceux vendus sous les dénominations commerciales de "CARBOPOL" (par exemple 934 et 940) par la Société "GOODRICH", des dérivés cellulosiques, tels que plus particulièrement une hydroxyéthylcellulose comme celle vendue sous la dénomination de "NATROSOL PLUS GRADE 330 CS" par la Société "AQUALON", une gomme de xanthane, la bentonite, une émulsion de copolymère acrylate d'ammonium/acrylamide réticulé, vendue sous la dénomination de "PAS 5161" par la Société "HOECHST" ; et des adjuvants tels que des huiles minérales, végétales, des esters d'acide gras, des alcools gras, des filtres ultraviolets, des pigments minéraux, tels que le dioxyde de titane, l'oxyde de zinc, l'oxyde de cérium et l'oxyde ferrique, des conservateurs, des parfums, des agents alcalinisants, des agents acidifiants, des agents stabilisants ou des colorants.

Enfin, l'invention porte sur l'utilisation de la composition telle que définie ci-dessus pour la protection de la peau ou des cheveux contre les radiations ultraviolettes et pour la pigmentation de la peau ou des cheveux.

Les compositions décrites ci-dessus peuvent également être utilisées comme composition de maquillage (fonds de teint, mascaras, fards à paupière, fards à joues) ou pour la coloration des cheveux.

Pour mieux faire comprendre l'objet de l'invention, on va en décrire maintenant, à titre d'exemples purement illustratifs et non-limitatifs, plusieurs modes de mise en oeuvre.

### EXEMPLE 1 : Crème solaire

### Première étape : Préparation d'une dispersion aqueuse de vésicules

On utilise une phase lipidique formulée comme suit :
- Lipide amphiphile non-ionique de formule : 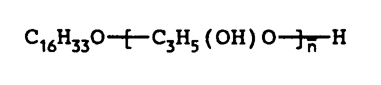 dans laquelle :
   · -C₃H₅(OH)O- est représenté par les structures suivantes, prises en mélange ou séparément : 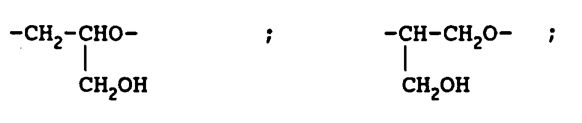
   · n̅ est une valeur statistique moyenne égale à 3 3,8 g
- Cholestérol 3,8 g
- Dicétylphosphate 0,4 g
On fond, en agitant doucement à une température de 100°C-110°C, le mélange ayant la formulation ci-dessus, et, dans le mélange fondu, on introduit progressivement 16 g d'eau portée à 95°C, sous agitation lente, après quoi on agite vivement dans une turbine munie de pales, jusqu'à l'obtention d'une masse gélifiée blanc cassé.

On ajoute alors 2 g de glycérine et 16,9 g d'eau à 90-95°C. On maintient la température et l'agitation pendant 10 minutes.

Quand la température atteint 25°C, on affine le mélange par un passage dans un homogénéiseur haute pression à 5x10⁷ Pa de type "RANNIE" ou "GAULIN".

On introduit ensuite 2 g d'eau renfermant un conservateur, puis on affine de nouveau à l'homogénéiseur haute pression.

Les vésicules ont un diamètre moyen de l'ordre de 300 nm.

### Deuxième étape : Préparation de la crème

A la dispersion aqueuse de vésicules obtenue à la piemière étape, on ajoute les ingrédients suivants :

| | |
|---|---|
| - Huile minérale | 15 g |
| - p-Méthoxycinnamate de 2-éthylhexyle commercialisé sous la dénomination de "PARSOL MCX" par la Société "GIVAUDAN" | 5 g |
| - Pigment mélanique obtenu par polymérisation oxydante du 5,6-dihydroxyindole en présence d'eau oxygénée en milieu ammoniacal | 0,1 g |
| - Oxyde de fer jaune | 0,04 g |
| - Oxyde de fer rouge | 0,05 g |
| - Oxyde de titane (anatase) | 3 g |
| - Acide polyacrylique réticulé (PM 4 000 000) commercialisé sous la dénomination de "CARBOPOL 940" par la Société "GOODRICH" (agent épaississant) | 0,42 g |
| - Triéthanolamine | 0,4 g |
| - Eau | 30 g |
| - Parfum, conservateurs | qs |

On introduit, dans la dispersion aqueuse de vésicules obtenue à la première étape, à 35°C, l'huile minérale, le p-méthoxycinnamate de 2-éthylhexyle et du parfum, et on agite en turbine. On affine dans un homogénéiseur haute pression à 500 bars. On additionne ensuite le pigment mélanique, puis on effectue un passage du mélange obtenu dans un broyeur de type "FRYMA" ou "DYNOMILL". On ajoute ensuite les oxydes métalliques et on effectue un nouveau passage au broyeur. On épaissit en dernier lieu le mélange dans une turbine à pales, en lui ajoutant un gel aqueux bien homogène constitué par l'agent épaississant dissous avec la triéthanolamine dans l'eau renfermant les conservateurs et le parfum.

La composition ainsi obtenue est mise en oeuvre par application topique à raison de 2 mg/cm² sur une zone de peau en une seule application. La zone de peau traitée est soumise à une irradiation ultraviolette (lampe OSRAM ULTRAVITALUX) pendant 10 minutes à raison de 1 m W/cm². On constate que, une heure après l'irradiation, aucun érythème n'est visible sur la zone de peau testée alors que la même irradiation sur une autre zone de peau du même sujet conduit à un érythème an l'absence de tout traitement préalable.

### EXEMPLE 2 : Crème autobronzante

### Première étape : Préparation d'une dispersion aqueuse de vésicules

On utilise une phase lipidique formulée comme suit :
- Lipide amphiphile non-ionique de formule : 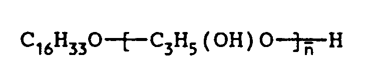 dans laquelle :
   · -C₃H₅(OH)O- est représenté par les structures suivantes, prises en mélange ou séparément : 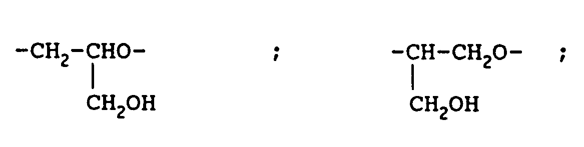
   · n̅ est une valeur statistique moyenne égale à 3 3,8 g
- Cholestérol 3,8 g
- Sel monosodique du glutamate de formule : 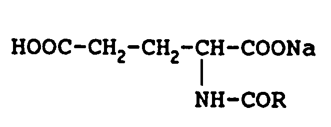 dans laquelle R est un mélange de radicaux alcényle et/ou alkyle en C₁₃-C₂₁ dérivé des acides gras du suif, commercialisé sous la dénomination de "ACYLGLUTAMATE HS 11" pas la Société "AJINOMOTO" 0,4 g
On fond, en agitant doucement à une température de 100°C-110°C, le mélange ayant la formulation ci-dessus, et, dans le mélange fondu, on introduit progressivement 15 g d'eau portée à 95°C, sous agitation lente, après quoi on agite vivement dans une turbine munie de pales, jusqu'à l'obtention d'une masse gélifiée blanc cassé.

On ajoute alors 5 g de glycérine et 13 g d'eau à 90-95°C. On maintient la température et l'agitation pendant 10 minutes.

Puis on abaisse la température à 60°C et on introduit 5 g de dihydroxyacétone en solution dans 3,8 g d'eau.

On affine le mélange obtenu par un passage dans un homogénéiseur haute pression à 500 bars de type "RANNIE" ou "GAULIN".

A 40°C, on introduit 0,8 g d'eau renfermant un conservateur, puis on affine de nouveau à l'homogénéiseur haute pression.

Les vésicules ont un diamètre moyen de l'ordre de 300 nm.

### Deuxième étape : Préparation de la crème

A la dispersion aqueuse de vésicules obtenue à la première étape, on ajoute les ingrédients suivants :

| | |
|---|---|
| - Huile minérale | 12 g |
| - Pigment mélanique obtenu par polymérisation oxydante du 5,6-dihydroxyindole en présence d'eau oxygénée en milieu ammoniacal | 0,1 g |
| - Oxyde de fer jaune | 0,15 g |
| - Oxyde de fer rouge | 0,1 g |
| - Oxyde de titane (anatase) | 2,2 g |
| - Hydroxyéthylcellulose commercialisée sous la dénomination de "NATROSOL PLUS GRADE 330 CS" par la Société "AQUALON" (agent épaississant) | 0,5 g |
| - Eau | 30 g |
| - Parfums, conservateur | qs |

On introduit, dans la dispersion aqueuse de vésicules obtenue à la première étape, à 35°C, l'huile minérale et du parfum, et on agite en turbine. On affine dans un homogénéiseur haute pression à 500 bars. On additionne ensuite le pigment mélanique ; puis on effectue un passage du mélange obtenu dans un broyeur de type "FRYMA" ou "DYNOMILL". On ajoute ensuite les oxydes métalliques et on effectue un nouveau passage au broyeur. On épaissit en dernier lieu le mélange dans une turbine à pales, en lui ajoutant un gel aqueux bien homogène constitué par l'agent épaississant dissous dans l'eau renfermant un conservateur et du parfum.

La composition ainsi obtenue est mise en oeuvre par application topique à raison de 2 mg/cm² sur une zone de peau en une seule application. Après quelques heures, la peau se colore en une couleur voisine de celle du bronzage naturel.

### EXEMPLE 3 : Lait solaire

On prépare une composition ayant la formulation suivante :
- Lipide amphiphile non-ionique de formule : 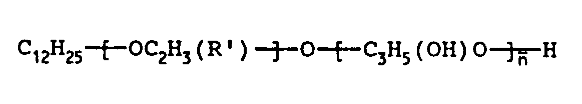 dans laquelle :
   · -C₃H₅(OH)O- est constitué par un mélange des radicaux : 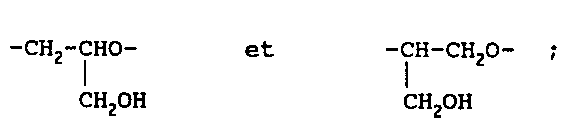
   · -OC₂H₃(R')- est constitué par un mélange des radicaux : 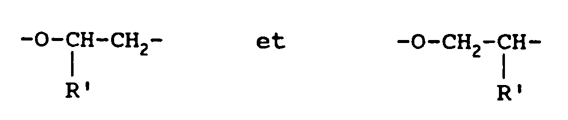
   · n̅ est une valeur statistique moyenne égale à 6
   · R' est un mélange des radicaux C₁₄H₂₉ et C₁₆H₃₃ 1,9 g
- Dimyristylphosphate 0,1 g
- Glycérine 1,5 g
- Huile de vaseline 15 g
- Pigment mélanique obtenu par polymérisation oxydante du 5,6-dihydroxyindole en présence d'eau oxygénée en milieu ammoniacal, sur du nitrure de bore commercialisé sous la dénomination de "SH P2" par la Société "KAWASAKI" 0,5 g
- Mélange d'un polydiméthylsiloxane hydroxylé en bout de chaîne et d'octaméthylcyclotétrasiloxane (13:87 en poids), commercialisé sous la dénomination de "Q2-1401" par la Société "DOW CORNING" 3,4 g
- Silicones volatiles 1,6 g
- 2-Ethylhexyl monococoate 3 g
- Acide polyacrylique réticulé (PM 4 000 000) commercialisé sous la dénomination de "CARBOPOL 940" par la Société "GOODRICH" 0,35 g
- Triéthanolamine 0,33 g
- Eau 76 g
- Conservateurs, parfum qs
On fond, en agitant doucement à une température de 85°C, un mélange du lipide non-ionique et du dimyristylphosphate. Dans le mélange fondu, on introduit progressivement 4 g d'eau portée à 85°C, sous agitation lente, après quoi on agite vivement dans une turbine munie de pales, jusqu'à l'obtention d'une masse gélifiée blanc cassé.

On ajoute alors sous agitation rapide la glycérine et 42 g d'eau à 60°C. On maintient la température et l'agitation pendant 10 minutes. Puis on ajoute l'huile de vaseline préalablement chauffée à 60°C.

Quand la température atteint 25°C, on affine le mélange par deux passages dans un homogénéiseur haute pression à 500 bars de type "RANNIE" ou "GAULIN".

On additionne ensuite le pigment mélanique sur nitrure de bore, puis on agite pendant 5 minutes. On ajoute alors le mélange des polysiloxanes, les silicones volatiles et le 2-éthylhexyl monococoate. On agite ensuite pendant 10 minutes.

On épaissit en dernier lieu le mélange dans une turbine à pales, en lui ajoutant un gel aqueux bien homogène constitué par l'agent épaississant dissous avec la triéthanolamine dans 30 g d'eau renfermant des conservateurs et du parfum.

La composition ainsi obtenue est mise en oeuvre comme dans l'exemple 1 . On observe les mêmes résultats.

### EXEMPLE 4 : Crème après-soleil

On prépare une composition selon la formulation suivante :
- Lipide non-ionique de formule : 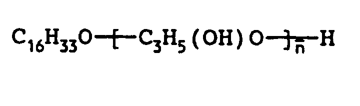 dans laquelle :
   · -C₃H₅(OH)O- est représenté par les structures suivantes, prises en mélange ou séparément : 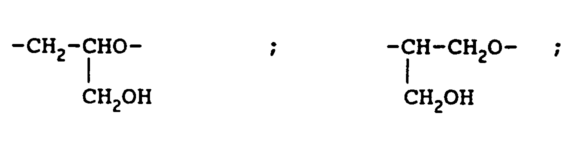 et
   · n̅ est une valeur statistique moyenne égale à 3 3,8 g
- Cholestérol 3,8 g
- Dicétylphosphate 0,4 g
- Glycérine 2 g
- Huile de vaseline 15 g
- Pigment mélanique obtenu par polymérisation oxydante du 5,6-dihydroxyindole en présence d'eau oxygénée en milieu ammoniacal, sur de l'oxychlorure de bismuth commercialisé sous la dénomination de "PEARL GLO UVR 1086" par la Société "MALLINCKRODT" 0,5 g
- Dérivé terpénique (bisabolol) commercialisé sous la dénomination de "DRAGOSANTOL" par la Société "DRAGOCO" 0,5 g
- Acide polyacrylique réticulé (PM 4 000 000) commercialisé sous la dénomination de "CARBOPOL 940" par la Société "GOODRICH" 0,42 g
- Triéthanolamine 0,4 g
- Eau 88 g
- Conservateur, parfum qs
On fond, en agitant doucement à une température de 100°C-110°C, un mélange du lipide non-ionique, du cholestérol et du dicétylphosphate. Dans le mélange fondu, on introduit progressivement 16 g d'eau portée à 95°C, sous agitation lente, après quoi on agite vivement dans une turbine munie de pales, jusqu'à l'obtention d'une masse gélifiée blanc cassé.

On ajoute alors la glycérine et 42 g d'eau à 60°C. On maintient la température et l'agitation pendant 10 minutes. Puis on ajoute l'huile de vaseline.

On affine le mélange obtenu par deux passages dans un homogénéiseur haute pression à 500 bars de type "RANNIE" ou "GAULIN".

On additionne ensuite le pigment mélanique sur oxychlorure de bismuth, et on agite pendant 5 minutes. On ajoute alors le dérivé terpénique.

On épaissit en dernier lieu le mélange dans une turbine à pales, en lui ajoutant un gel aqueux bien homogène constitué par l'agent épaississant dissous dans 30 g d'eau renfermant un conservateur et du parfum, et neutralisé par la triéthanolamine.

La composition ainsi obtenue est mise en oeuvre par application topique à raison de 2 mg/cm² sur une zone de peau préalablement exposée au soleil. Outre sa fonction apaisante, cette crème confère un léger hâle de la peau.

### EXEMPLE 5 : Crème solaire

On prépare la formulation suivante :
- Lipide non-ionique de formule : 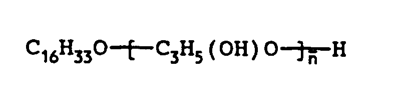 dans laquelle :
   · -C₃H₅(OH)O- est représenté par les structures suivantes, prises en mélange ou séparément : 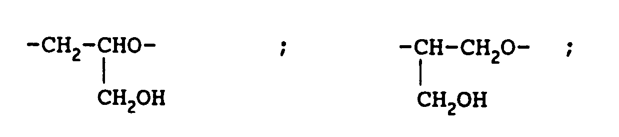 et
   · n̅ est une valeur statistique moyenne égale à 3 3,8 g
- Cholestérol 3,8 g
- Dicétylphosphate 0,4 g
- Glycérine 2 g
- Huile de vaseline 14,4 g
- Pigment mélanique obtenu par polymérisation oxydante du 5,6-dihydroxyindole en présence d'eau oxygénée en milieu ammoniacal sur du mica commercialisé sous la dénomination de "MICA CONCORD 1000" par la Société "SCIAMA" 0,5 g
- Oxyde de fer jaune 0,04 g
- Oxyde de fer sienne 0,05 g
- Acide polyacrylique réticulé (PM 4 000 000) commercialisé sous la dénomination de "CARBOPOL 940" par la Société "GOODRICH" 0,42 g
- Triéthanolamine 0,4 g
- Eau 74 g
- Conservateur, parfum qs
On fond, en agitant doucement à une température de 100°C-110°C, un mélange du lipide non-ionique, du cholestérol et du dicétylphosphate. Dans le mélange fondu, on introduit progressivement 16 g d'eau portée à 85°C, sous agitation lente, après quoi on agite vivement dans une turbine munie de pales, jusqu'à l'obtention d'une masse gélifiée blanc cassé.

On ajoute alors la glycérine et 28 g d'eau à 60°C. On maintient la température et l'agitation pendant 10 minutes. Puis on ajoute l'huile de vaseline préalablement chauffée à 60°C.

On affine le mélange obtenu par deux passages dans un homogénéiseur haute pression à 500 bars de type "RANNIE" ou "GAULIN".

On additionne ensuite sous agitation le pigment mélanique sur mica, puis on ajoute les oxydes de fer sous agitation.

On épaissit en dernier lieu le mélange dans une turbine à pales, en lui ajoutant un gel aqueux bien homogène constitué par l'agent épaississant dissous dans 30 g d'eau renfermant un conservateur et du parfum, et neutralisé par la triéthanolamine.

La composition ainsi obtenue est mise en oeuvre selon le procédé décrit à l'exemple 1. On observe les mêmes résultats.

### EXEMPLE 6 : Crème antisolaire

On prépare une composition antisolaire de la façon suivante :
Dans une première étape, on fait fondre, en agitant doucement, à une température de 85°C, 3,2 g de phytostérol polyoxyéthyléné à 5 moles d'oxyde d'éthylène vendu par la société NIKKO sous la dénomination commerciale "GENEROL 122 E5". Puis au mélange fondu, on ajoute 4,8 g de lécithine hydrogénée à 30-35 % de phosphatidylcholine hydrogénée vendu par la société NIKKO sous la dénomination commerciale "LECINOL S10", et ceci jusqu'à homogénéisation parfaite (5 minutes).

On introduit dans le mélange fondu 24 g d'eau portée à 80°C et contenant un conservateur et l'on mélange pendant environ 5 minutes ; on laisse ensuite gonfler le mélange pendant 1 heure. A la phase ainsi obtenue, on ajoute 36 g d'eau à 20°C ; on agite le mélange quelques minutes et on affine le mélange par un passage dans un homogénéiseur haute pression à 5x10⁷ Pa de type "RANNIE" ou "GAULIN" avant de laisser revenir le mélange à température ambiante.

Dans une seconde étape, on ajoute un mélange de 0,5 g de pigment mélanique obtenu par oxydation du 5,6-dihydroxyindole et de 10 g d'huile de vaseline, en agitant à l'"ULTRA TURRAX".

On prépare un gel aqueux bien homogène constitué de 0,4 g d'agent épaississant vendu sous la dénomination commerciale "CARBOPOL 940" par la société GOODRICH dissous dans 8 g d'eau renfermant un conservateur. Dans ce gel, on ajoute 10 g d'oxyde de cérium vendu sous la dénomination commerciale "OPALINE BROYEE" par la société RHONE POULENC. On ajoute ensuite ce gel à la dispersion de vésicules, puis on complète à 100 g avec de l'eau. Le pH est ajusté à 6,5 avec de la triéthanolamine.

La composition ainsi obtenue est mise en oeuvre selon le procédé décrit à l'exemple 1. On observe les mêmes résultats.

### EXEMPLE 7 : Crème de protection de la peau

On; prépare une crème de protection de la peau de la façon suivante :
Dans une première étape, on fait fondre, en agitant doucement à une température de 100°C-110°C, un mélange de 3,8 g de lipide non-ionique de formule : 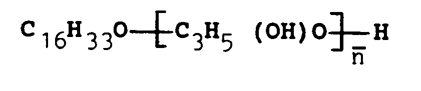
formule dans laquelle :
n̅ est une valeur statistique moyenne égale à 3 et -C₃H₅(OH)O - est représenté par les structures suivantes prises en mélange ou séparément : 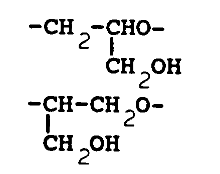
avec 3,8g de cholestérol et 0,4 g de dicétylphosphate.

On introduit progressivement dans le mélange fondu, 16 g d'eau portée à 85°C contenant un conservateur et l'on mélange pendant environ 5 minutes. A la phase ainsi obtenue, on ajoute 24 g d'eau à 20°C. On agite le mélange pendant quelques minutes.

On affine le mélange obtenu par deux passages dans un homogénéiseur haute pression 5x10⁷ Pa de type "RANNIE" ou "GAULIN".

On ajoute ensuite un mélange de 0,002 g de mélanine de seiche et de 15 g d'huile de vaseline. On agite ensuite pendant 5 minutes.

On prépare un gel aqueux bien homogène constitué de 0,42 g d'agent épaississant vendu sous la dénomination commerciale "CARBOPOL 940" par la société GOODRICH, dissous dans 30 g d'eau renfermant un conservateur. Dans ce gel, on ajoute 2 g d'oxyde de titane vendu sous la dénomination commerciale "P 25" par la société DEGUSSA. On ajoute ensuite ce gel à la dispersion de vésicules, puis on complète à 100 g avec de l'eau. Le pH est ajusté à 7 avec de la triéthanolamine.

La composition ainsi obtenue est mise en oeuvre par application topique. Elle confère à la peau une grande douceur et un léger hâle.

### EXEMPLE 8 (Comparatif)

On a préparé, à titre comparatif, deux types de composition.

### Composition A₁ (ne faisant pas partie de l'invention)

La composition A₁ a été préparée selon le procédé décrit à l'exemple 2 de EP-A 0 386 380 de façon à contenir de la mélanine à l'intérieur des vésicules en ajoutant la mélanine avant la formation des vésicules.

La composition A₁ a la formulation suivante (en poids) :
- Phase lipidique 8 % constituée de :
   · α - phosphatidylcholine 6,26 %
   · Dicétylphosphate 1,29 %
   · Cholestérol 0,45 %
- Mélanine synthétique "SIGMA" obtenue par oxydation de la tyrosine à l'aide d'un persulfate ayant une granulométrie inférieure à 1 µm 0,1 %
- Agent épaississant vendu sous la dénomination commerciale "CARBOPOL 940" par la société GOODRICH 0,3 %
- Eau qsp 100 %

### Composition A₂

Cette composition est identique à la composition A₁, sauf qu'elle ne contient pas de mélanine.

### Composition B₁ (selon l'invention)

La composition B₁ a été préparée comme décrit dans l'exemple 1 en ajoutant la mélanine après la formation des vésicules dans la phase externe de dispersion des vésicules.

La composition B₁ a la formulation suivante (en poids) :
- Phase lipidique 8 %
   constituée de :
   · Lipide amphiphile 6,26 %
      non-ionique de formule : 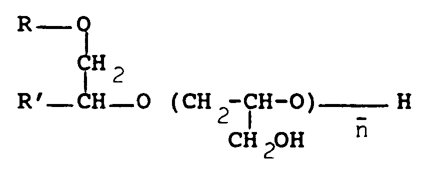 dans laquelle :
      R est le radical C₁₂H₂₅
      R' est un mélange de radicaux C₁₄H₂₉ et C₁₆H₃₃
      n̅ est une valeur statistique égale à 6
   · Sel monosodique de glutamate vendu sous la dénomination commerciale "ACYLGLUTAMATE HS 11" par la société AJINOMOTO 0,8 %
- Pigment mélanique obtenu par polymérisation oxydante du 5,6 dihydroxyindole en présence d'eau oxygénée en milieu ammoniacal ayant une granulométrie de 15 µm 0,1 %
- Agent épaississant vendu sous la dénomination commerciale "CARBOPOL 940" par la société GOODRICH 0,3 %
- Eau qsp 100 %

La composition B₂ est identique à la composition B₁ sauf qu'elle ne contient pas de mélamine.

### Essais

On a mélangé les compositions A₁, A₂, B₁, B₂ , avec 10 % en poids d'acide linoléique et on a soumis les mélanges obtenus au "sun test" pendant 7 heures. On a mesuré l'absorption au spectrophotomètre à 233 nm (qui est fonction de la quantité d'hydroperoxydes diéniques présents au temps To avant le "sun test" et au temps T7 après le "sun test") ; les résultats obtenus sont donnés dans le tableau ci-dessous :

| Produit | To | T7 | Différence T7 - To |
|---|---|---|---|
| A₂ | 0,84 | 1,21 | 0,37 |
| A₁ | 1,55 | 1,69 | 0,14 |
| B₂ | 1,16 | 1,24 | 0,08 |
| B₁ | 0,97 | 0,95 | 0,02 |

Ces résultats montrent que la composition B₁ selon l'invention inhibe la peroxydation des corps gras insaturés.

### Exemple 9 : Fond de teint protecteur de l'épiderme.

En opérant comme dans l'exemple 5, on prépare une composition ayant la formulation suivante:

| | |
|---|---|
| - Alcool cétylique polyglycérolé à 3 moles de glycérol | 2,7 g |
| - Cholestérol | 2,7 g |
| - Glycérine | 2 g |
| - Lactate de monométhylsilanol dans l'eau à 1% | 5 g |
| - Isoparaffine | 8 g |
| - Cyclopentadiméthylsiloxane | 6 g |
| - Huile de sésame | 8 g |
| - Fraction liquide de beurre de karité | 2 g |
| - Mélange d'acide palmitoylcollagénique de bovidés, acide palmitique, palmitate d'isopropyle (60/20/12 en poids) | 0,6 g |
| - Pigment mélanique obtenu par polymérisation oxydante du 5,6 dihydroxyindole en présence d'eau oxygénée en milieu ammoniacal (granulométrie : 15 µ). | 0,1 g |
| - Oxyde de fer jaune | 0,5 g |
| - Oxyde de fer brun et jaune | 0,32 g |
| - Oxyde de fer noir | 0,15 g |
| - Oxyde de titane | 3,03 g |
| - Paraméthoxycinnamate 2-éthylhexyle | 1 g |
| - Triéthanolamine | 0,5 g |
| - Polymère carboxyvinylique synthétisé dans l'acétate éthyle | 0,5 g |
| - Eau déminéralisée stérilisée | 62,3 g |

La composition ainsi obtenue est appliquée sur la peau du visage. Elle confère à la peau une grande douceur et un aspect légèrement hâlé.

## Revendications

1. Composition cosmétique comportant en dispersion aqueuse des vésicules lipidiques à structure lamellaire encapsulant une phase aqueuse, caractérisée par le fait qu'elle contient au moins un pigment mélanique de granulométrie comprise entre 500 et 50.000 nm, en mélange avec les vésicules dans la phase externe de la dispersion.

2. Composition selon la revendication 1 , caractérisée par le fait que le (ou les) pigment (s) mélanique (s) sont obtenus par oxydation d'au moins un composé indolique, ou par extraction de la mélanine a partir de substances en contenant.

3. Composition selon la revendication 2 caractérisée par le fait que le composé indolique est choisi parmi ceux répondant à la formule (I) : 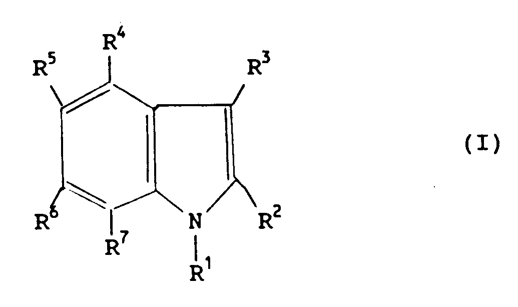 dans laquelle :
- R¹ et R³ représentent, indépendamment l'un de l'autre, hydrogène ou alkyle en C₁-C₄ ;
- R² représente hydrogène, alkyle en C₁-C₄, carboxyle ou (alcoxy en C₁-C₄)-carbonyle ;
- R⁴ et R⁷ représentent, indépendamment l'un de l'autre, hydrogène, hydroxy, alkyle en C₁-C₄, amino, alcoxy en C₁-C₄, (acyl en C₂-C₄)-oxy, (acyl en C₂-C₄)-amino ;
- R⁵ représente hydrogène, hydroxy, alcoxy en C₁-C₄, alkyle en C₁-C₄, halogène, amino, (acyl en C₂-C₁₄)-oxy, (acyl en C₂-C₄)-amino, triméthylsilyloxy ;
- R⁶ représente hydrogène, hydroxy, alcoxy en C₁-C₄, amino, (acyl en C₂-C₄)-oxy, (acyl en C₂-C₄)-amino, triméthylsilyloxy, (hydroxyalkyl en C₂-C₄)-amino ;
- R⁵ et R⁶ pouvant également former, conjointement avec les atomes de carbone auxquels ils sont rattachés, un cycle méthylènedioxy éventuellement substitué par un groupement alkyle en C₁-C₄ ou alcoxy en C₁-C₄, ou bien un cycle carbonyldioxy :
- au moins l'un des radicaux R⁴ à R⁷ représente un groupement OZ ou NHR⁰ avec au plus un des radicaux R⁴ à R⁷ représentant NHR⁰ ; et au plus deux des radicaux R⁴ à R⁷ représentent OZ et, dans le cas où Z représente hydrogène, les deux OH sont dans les positions 5 et 6 ; et au moins l'un des radicaux R⁴ à R⁷ représente hydrogène, et dans le cas où un seul de ces radicaux représente hydrogène, un seul radical parmi R⁴ à R⁷ représente alors NHR⁰ ou OZ, les autres radicaux représentant alkyle en C₁-C₄ ;
- le R⁰ du groupement NHR⁰ désignant hydrogène, acyle en C₂-C₄, hydroxyalkyle en C₂-C₄, et le Z du groupement OZ désignant hydrogène, acyle en C₂-C₁₄, alkyle en C₁-C₄, ou triméthylsilyle,
et leurs sels de métaux alcalins, alcalino-terreux, d'ammonium ou d'amines.

4. Composition selon la revendication 3, caractérisée par le fait que le composé indolique est choisi dans le groupe formé par le 4-hydroxyindole, le 5-hydroxyindole, le 6-hydroxyindole, le 7-hydroxyindole, le 4-hydroxy 5-méthoxyindole, le 4-hydroxy 5-éthoxyindole, le 2-carboxyl 5-hydroxyindole, le 5-hydroxy 6-méthoxyindole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-dihydroxyindole, le 3-méthyl 5,6-diydroxyindole, le 2,3-diméthyl 5,6-dihydroxyindole, le 2-carboxyl 5,6-dihydroxyindole, le 4-hydroxy 5-méthyl indole, le 2-carboxyl 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxyl 5-méthylindole, le 6-hydroxy 2-éthoxycarbonyl 5-méthoxyindole, le 6-N-β-hydroxyéthylaminoindole, le 4-aminoindole, le 5-aminoindole, le 6-aminoindole, le 7-aminoindole, le N-méthyl 6β-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6-diacétoxyindole, le 5-méthoxy 6-acétoxyindole, le 5,6-diméthoxyindole, le 5,6-dibenzyloxyindole, le 5,6-méthylènedioxyindole, le 5,6- triméthylsilyloxyindole, l'ester phosphorique du 5,6-dihydroxyindole et les sels d'addition de ces composés.

5. Composition selon l'une des revendications 1 à 4 caracterisée par le fait que le (ou les) pigment(s) mélanique(s) est (ou sont) associé(s) à au moins une charge particulaire.

6. Composition selon la revendication 5 caractérisée par le fait que le (ou les) pigment(s) mélanique(s) résulte(nt) de l'oxydation d'au moins un composé indolique de la formule (I), telle que définie à l'une des revendications 3 et 4, en mélange avec la charge dans un milieu essentiellement non-solvant de ladite charge, ou de la polymérisation oxydante de précurseurs mélaniques sur la charge.

7. Composition selon l'une des revendications 5 et 6 caractérisée par le fait que la charge particulaire est une charge minérale inerte, de granulométrie inférieure à 20 000 nm.

8. Composition selon l'une des revendications 5 et 6 caractérisée par le fait que la charge particulaire est une charge polymérique inerte, de granulométrie inférieure à 100 000 nm, choisie parmi les polymères naturels ou synthétiques, organiques ou inorganiques, à réseau réticulé, cristallin ou amorphe, ayant un poids moléculaire compris entre 5000 et 5 000 000.

9. Composition selon la revendication 8, caractérisée par le fait que les polymères sont choisis dans le groupe formé par les polymères dérivés de la kératine, de la chitine, de la cellulose, les polyamides et les homo- ou copolymères résultant de la polymérisation de monomères mono- ou polyéthyléniques, aliphatiques ou aromatiques, à réseau réticulé, cristallin ou amorphe.

10. Composition selon la revendication 9 caractérisée par le fait que les polymères dérivés de la kératine sont choisis dans le groupe formé par les kératines animales ou humaines ; les kératines modifiées chimiquement, par hydrolyse ou oxydation, ayant un poids moléculaire compris entre 10 000 et 250 000, et la fibroïne de soie.

11. Composition selon la revendication 9, caractérisée par le fait que les polymères dérivés de la chitine sont constitués par la chitine et/ou son dérivé désacétylé.

12. Composition selon la revendication 9, caractérisée par le fait que les polymères cellulosiques sont des celluloses microcristallines.

13. Composition selon la revendication 9, caractérisée par le fait que les polymères synthétiques sont choisis dans le groupe formé par le polyéthylène, le polypropylène, le polystyrène, le poly(méthacrylate de méthyle) et le poly(méthacrylate de méthyle) réticulé.

14. Composition selon la revendication 9, caractérisée par le fait que le polymère est une poly-β-alanine réticulée.

15. Composition selon la revendication 8, caractérisée par le fait que les polymères sont constitués par des microsphères creuses de copolymère de chlorure de vinylidène et d'acrylonitrile ou par des microsphères poreuses de polyamide 12, de polyamide 6 ou de copolyamide 6/12.

16. Composition selon la revendication 8, caractérisée par le fait que les polymères sont des microéponges constituées de polymères réticulés de styrène/divinylbenzène, de méthacrylate de méthyle/diméthacrylate d'éthylèneglycol ou de stéarate de vinyle/divinylbenzène

17. Composition selon la revendication 8, caractérisée par le fait que les polymères sont constitués par des poudres de silicone.

18. Composition selon l'une des revendications 5 et 6, caractérisée par le fait que la charge particulaire est une charge constituée par des particules à structure lamellaire, organiques ou minérales, ayant une dimension inférieure à 50 000 nm.

19. Composition selon la revendication 18 caractérisée par le fait que les particules à structure lamellaire sont choisies parmi la L-lauroyllysine, les microparticules de céramique éventuellement recouvertes de poudre de zirconium, le dioxyde de titane lamellaire, le talc lamellaire, le nitrure de bore, le mica lamellaire, l'oxychlorure de bismuth.

20. Composition selon l'une des revendications 18 et 19, caractérisée par le fait que les particules à structure lamellaire sont constituées de feuillets pour lesquels le rapport entre la plus grande dimension et l'épaisseur est compris entre 2 et 100.

21. Composition selon l'une des revendications 1 à 20, caractérisée par le fait que le (ou les) lipide(s) constitutif(s) de la phase lipidique des vésicules est (ou sont) choisi(s) dans le groupe formé par les lipides amphiphiles non-ioniques, les lipides amphiphiles ioniques et leurs mélanges.

22. Composition selon la revendication 21, caractérisée par le fait que les lipides amphiphiles nonioniques sont choisis dans le groupe formé par
(1) les éthers de polyglycérol, linéaires ou ramifiés, de formule : 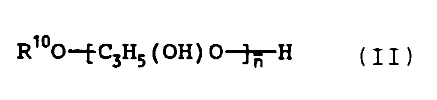 dans laquelle :
· -C₃H₅(OH)O est représenté par les structures suivantes prises en mélange ou séparément : -CH₂CHOHCH₂O- ; 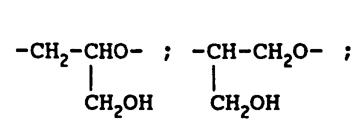
· n̅ est une valeur statistique moyenne comprise entre 2 et 6 ;
· R¹⁰ représente :
(a) une chaîne aliphatique, linéaire ou ramifiée, contenant de 12 à 18 atomes carbone ;
(b) un reste R¹¹CO, où R¹¹ est un radical aliphatique, linéaire ou ramifié, en C₁₁-C₁₇ ;
(c) un reste R¹²-[-OC₂H₃(R¹³)-]-, où :
· R¹² peut prendre la signification (a) ou (b) donnée pour R¹⁰ ;
· OC₂H₃(R¹³)- est représenté par les structures suivantes, prises en mélange ou séparément : 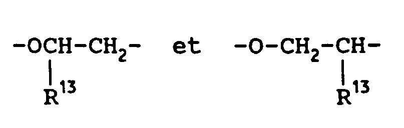 où R¹³ prend la signification (a) donnée pour R¹⁰ ;
(2) les alcools gras polyoxyéthylénés ;
(3) les esters de polyols éventuellement polyoxyéthylénés ;
(4) les cérébrosides ; et
(5) le stéarate de polyglycérol oxyéthyléné.

23. Composition selon l'une des revendications 21 à 22 caractérisée par le fait qu'aux lipides sont associés au moins un additif stabilisant choisi dans le groupe formé par les stérols et les stabilisants anioniques.

24. Composition selon l'une des revendications 21 à 23 caractérisée par le fait que les vésicules ont un diamètre moyen compris entre 10 et 1000 nm.

25. Composition selon l'une des revendications 1 à 24 caractérisée par le fait que la phase lipidique des vésicules, constituée par le (ou les) lipide(s) et le (ou les) stabilisant(s) éventuellement associé(s) à ceux-ci, représente de 0,1 à 16% du poids total de la composition, la proportion du (ou des) stabilisant(s) ne dépassant pas 10% du poids total de la composition.

26. Composition selon l'une des revendications 1 à 25 caractérisée par le fait que les vésicules sont présentes dans des proportions comprises entre 0,5 et 15 % en poids par rapport au poids total de la composition.

27. Composition selon l'une des revendications 1 à 26 caractérisée par le fait que le (ou les) pigment(s) mélanique(s), éventuellement sur charge, est (ou sont) présent(s) dans des proportions comprises entre 0,01 et 5 % en poids par rapport au poids total de la composition.

28. Composition selon l'une des revendications 1 à 27, caractérisée par le fait qu'elle se présente sous forme de lotion, de gel, de crème et de lait.

29. Composition selon l'une des revendications 1 à 28 caractérisée par le fait qu'elle contient au moins un additif cosmétiquement acceptable choisi dans le groupe formé par la dihydroxyacétone, les silicones, les agents épaississants, les huiles minérales, les huiles végétales, les esters d'acide gras, les alcools gras, les filtres ultraviolets, les pigments minéraux, les conservateurs, les parfums, les agents alcalinisants, les agents acidifiants, les agents stabilisants et les colorants.

30. Utilisation cosmétique de la composition selon l'une des revendications 1 à 29 pour la protection de la peau ou des cheveux contre les radiations ultraviolettes, ou pour la pigmentation de la peau ou des cheveux.

31. Utilisation cosmétique de la composition selon l'une des revendications 1 à 29 pour le maquillage ou pour la coloration des cheveux.

## Claims

1. Cosmetic composition, comprising in an aqueous dispersion, lipid vesicles with a lamellar structure encapsulating an aqueous phase, characterized by the fact that it contains at least one melanin pigment having a particle size of between 500 and 50,000 nm, mixed with the vesicles in the outer phase of the dispersion.

2. Composition according to Claim 1, characterized by the fact that the melanin pigment or pigments are obtained by oxidation of at least one indole compound, or by extraction of the melanin from substances containing it.

3. Composition according to Claim 2, characterized by the fact that the indole compound is chosen from those corresponding to the formula (I): 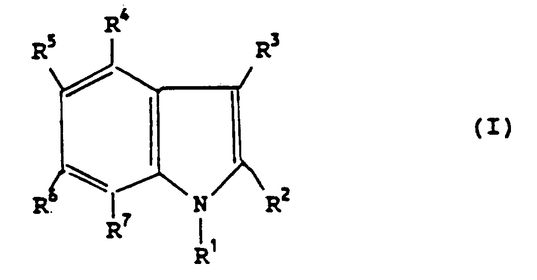 in which:
- R¹ and R³ represent, independently of each other, hydrogen or C₁-C₄ alkyl;
- R² represents hydrogen, C₁-C₄ alkyl, carboxyl or (C₁-C₄ alkoxy)-carbonyl;
- R⁴ and R⁷ represent, independently of each other, hydrogen, hydroxy, C₁-C₄ alkyl, amino, C₁-C₄ alkoxy, (C₂-C₄ acyl)-oxy, (C₂-C₄ acyl)-amino;
- R⁵ represents hydrogen, hydroxy, C₁-C₄ alkoxy, C₁-C₄ alkyl, halogen, amino, (C₂-C₁₄ acyl)-oxy, (C₂-C₄ acyl)-amino, trimethylsilyloxy;
- R⁶ represents hydrogen, hydroxy, C₁-C₄ alkoxy, amino, (C₂-C₄ acyl)-oxy, (C₂-C₄ acyl)-amino, trimethylsilyloxy, (C₂-C₄ hydroxyalkyl)-amino;
- R⁵ and R⁶ being also capable of forming, together with the carbon atoms to which they are attached, a methylenedioxy ring optionally substituted by a C₁-C₄ alkyl or C₁-C₄ alkoxy group, or alternatively a carbonyldioxy ring:
- at least one of the radicals R⁴ to R⁷ represents an OZ or NHR⁰ group with not more than one of the R⁴ to R⁷ radicals representing NHR⁰; and not more than two of the radicals R⁴ to R⁷ represent OZ and, in the case where Z represents hydrogen, the two OH groups are in positions 5 and 6; and at least one of the radicals R⁴ to R⁷ represents hydrogen, and in the case where only one of these radicals represents hydrogen, only one radical from among R⁴ to R⁷ then represents NHR⁰ or OZ, the other radicals representing C₁-C₄ alkyl;
- the R⁰ of the NHR⁰ groups denoting hydrogen, C₂-C₄ acyl, C₂-C₄ hydroxyalkyl, and the Z of the OZ group denoting hydrogen, C₂-C₁₄ acyl, C₁-C₄ alkyl or trimethylsilyl, and their alkali metal, alkaline-earth metal, ammonium or amine salts.

4. Composition according to Claim 3, characterized by the fact that the indole compound is chosen from the group composed of 4-hydroxyindole, 5-hydroxyindole, 6-hydroxyindole, 7-hydroxyindole, 4-hydroxy-5-methoxyindole, 4-hydroxy-ethoxyindole, 2-carboxy-5-hydroxyindole, 5-hydroxy-6-methoxyindole, 6-hydroxy-7-methoxyindole, 5-methoxy-6-hydroxyindole, 5,6-dihydroxyindole, N-methyl-5,6-dihydroxyindole, 2-methyl-5,6-dihydroxyindole, 3-methyl-5,6-dihydroxyindole, 2,3-dimethyl-5,6-dihydroxyindole, 2-carboxy-5,6-dihydroxyindole, 4-hydroxy-5-methylindole, 2-carboxy-6-hydroxyindole, 6-hydroxy-N-methylindole, 2-ethoxycarbonyl-5,6-dihydroxyindole, 4-hydroxy-7-methoxy-2,3-dimethylindole, 4-hydroxy-5-ethoxy-N-methylindole, 6-hydroxy-5-methoxy-2-methylindole, 6-hydroxy-5-methoxy-2,3-dimethylindole, 6-hydroxy-2-ethoxycarbonylindole, 7-hydroxy-3-methylindole, 5-hydroxy-6-methoxy-2,3-dimethylindole, 5-hydroxy-3-methylindole, 5-acetoxy-6-hydroxyindole, 5-hydroxy-2-ethoxycarbonylindole, 6-hydroxy-2-carboxy-5-methylindole, 6-hydroxy-2-ethoxycarbonyl-5-methoxyindole, 6-N-β-hydroxyethylaminoindole, 4-aminoindole, 5-aminoindole, 6-aminoindole, 7-aminoindole, N-methyl-6β-hydroxyethylaminoindole, 6-amino-2,3-dimethylindole, 6-amino-2,3,4,5-tetramethylindole, 6-amino-2,3,4-trimethylindole, 6-amino-2,3,5-trimethylindole, 6-amino2,3,6-trimethylindole, 5,6-diacetoxyindole, 5-methoxy-6-acetoxyindole, 5,6-dimethoxyindole, 5,6-dibenzyloxyindole, 5,6-methylenedioxyindole, 5,6-trimethylsilyloxyindole, 5,6-dihydroxyindole phosphoric ester and the addition salts of these compounds.

5. Composition according to one of Claims 1 to 4, characterized by the fact that the melanin pigment or pigments is (or are) combined with at least one particulate filler.

6. Composition according to Claim 5, characterized by the fact that the melanin pigment or pigments results or result from the oxidation of at least one indole compound of formula (I), as defined in one of Claims 3 and 4, mixed with the filler in an essentially non-solvent medium for the said filler, or from the oxidative polymerization of melanin precursors on the filler.

7. Composition according to one of Claims 5 and 6, characterized by the fact that the particulate filler is an inert mineral filler with a particle size of less than 20,000 nm.

8. Composition according to one of Claims 5 and 6, characterized by the fact that the particulate filler is an inert polymeric filler, with a particle size of less than 100,000 nm, chosen from natural or synthetic, organic or inorganic, cross-linked, crystalline or amorphous polymers, having a molecular weight of between 5000 and 5,000,000.

9. Composition according to Claim 8, characterized by the fact that the polymers are chosen from the group composed of polymers derived from keratin, chitin, cellulose, polyamides and homo- or copolymers resulting from the polymerization of mono- or polyethylenic, aliphatic or aromatic, cross-linked, crystalline or amorphous monomers.

10. Composition according to Claim 9, characterized by the fact that the polymers derived from keratin are chosen from the group composed of animal or human keratins, modified chemically, by hydrolysis or oxidation, having a molecular weight of between 10,000 and 250,000, and silk fibroin.

11. Composition according to Claim 9, characterized by the fact that the polymers derived from chitin consist of chitin and/or its deacetylated derivative.

12. Composition according to Claim 9, characterized by the fact that the cellulosic polymers are microcrystalline celluloses.

13. Composition according to Claim 9, characterized by the fact that the synthetic polymers are chosen from the group composed of polyethylene, polypropylene, polystyrene, poly(methyl methacrylate) and cross-linked poly(methyl methacrylate).

14. Composition according to Claim 9, characterized by the fact that the polymer is a cross-linked poly-β-alanine.

15. Composition according to Claim 8, characterized by the fact that the polymers consist of hollow microspheres of vinylidene chloride and acrylonitrile copolymer or of porous microspheres of polyamide 12, polyamide 6 or copolyamide 6/12.

16. Composition according to Claim 8, characterized by the fact that the polymers are microsponges consisting of cross-linked polymers of styrene/divinylbenzene, methyl methacrylate/ethylene glycol dimethacrylate or vinyl stearate/divinylbenzene.

17. Composition according to Claim 8, characterized by the fact that the polymers consist of silicone powders.

18. Composition according to one of Claims 5 and 6, characterized by the fact that the particulate filler is a filler consisting of organic or mineral particles with a lamellar structure, having a size of less than 50,000 nm.

19. Composition according to Claim 18, characterized by the fact that the particles with a lamellar structure are chosen from L-lauroyllysine, microparticles of ceramic which are optionally coated with zirconium powder, lamellar titanium dioxide, lamellar talc, boron nitride, lamellar mica and bismuth oxychloride.

20. Composition according to one of Claims 18 and 19, characterized by the fact that the particles with a lamellar structure consist of layers for which the ratio between the largest size and the thickness is between 2 and 100.

21. Composition according to one of Claims 1 to 20, characterized by the fact that the constituent lipid or lipids of the lipid phase of the vesicles is (or are) chosen from the group composed of non-ionic amphiphilic lipids, ionic amphiphilic lipids and mixtures thereof.

22. Composition according to Claim 21, characterized by the fact that the non-ionic amphiphilic lipids are chosen from the group composed of
(1) linear or branched polyglycerol esters of formula: 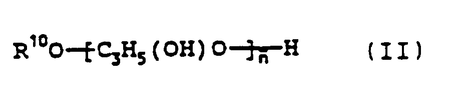 in which:
· -C₃H₅(OH)O is represented by the following structures taken in combination or separately: -CH₂CHOHCH₂O; 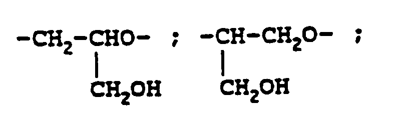
· n̅ is a mean statistical value between 2 and 6;
· R¹⁰ represents:
(a) a linear or branched aliphatic chain containing 12 to 18 carbon atoms;
(b) a residue R¹¹CO, where R¹¹ is a linear or branched aliphatic C₁₁-C₁₇ radical;
(c) a residue R¹²-[-OC₂H₃(R¹³)-]-, where:
· R¹² can have the meaning (a) or (b) given for R¹⁰;
· OC₂H₃(R¹³)- is represented by the following structures, taken in combination or separately: 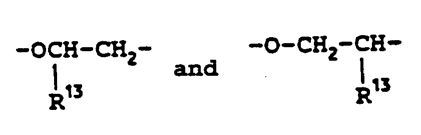 where R¹³ has the meaning (a) given for R¹⁰;
(2) polyoxyethylenated fatty alcohols;
(3) optionally polyoxyethylenated polyol esters;
(4) cerebrosides; and
(5) oxyethylenated polyglycerol stearate.

23. Composition according to one of Claims 21 to 22, characterized by the fact that at least one stabilizing additive chosen from the group composed of sterols and anionic stabilizers is combined with the lipids.

24. Composition according to one of Claims 21 to 23, characterized by the fact that the vesicles have a mean diameter of between 10 and 1000 nm.

25. Composition according to one of Claims 1 to 24, characterized by the fact that the lipid phase of the vesicles, which consists of the lipid or lipids and the stabilizer or stabilizers optionally combined therewith, represents 0.1 to 16 % of the total weight of the composition, the proportion of the stabilizer or stabilizers not exceeding 10 % of the total weight of the composition.

26. Composition according to one of Claims 1 to 25, characterized by the fact that the vesicles are present in proportions of between 0.5 and 15 % by weight relative to the total weight of the composition.

27. Composition according to one of Claims 1 to 26, characterized by the fact that the melanin pigment or pigments, optionally on a filler, is (or are) present in proportions of between 0.01 and 5 % by weight relative to the total weight of the composition.

28. Composition according to one of Claims 1 to 27, characterized by the fact that it is in the form of a lotion, a gel, a cream and a milk.

29. Composition according to one of Claims 1 to 28, characterized by the fact that it contains at least one cosmetically acceptable additive chosen from the group composed of dihydroxyacetone, silicones, thickening agents, mineral oils, vegetable oils, fatty acid esters, fatty alcohols, ultraviolet-screening agents, mineral pigments, preservatives, perfumes, alkalinizing agents, acidifying agents, stabilizing agents and colorants.

30. Cosmetic use of the composition according to one of Claims 1 to 29, for protecting the skin or hair against ultraviolet radiation, or for pigmenting the skin or hair.

31. Cosmetic use of the composition according to one of Claims 1 to 29, as make-up or for dyeing the hair.

## Patentansprüche

1. Kosmetisches Mittel, umfassend in wäßriger Dispersion Lipidvesikel mit lamellarer Struktur, welche eine wäßrige Phase einkapseln, dadurch gekennzeichnet, daß es wenigstens ein Melaninpigment mit einer Korngröße im Bereich von 500 bis 50 000 nm in Mischung mit den Vesikeln in der externen Phase der Dispersion enthält.

2. Mittel nach Anspruch 1, dadurch gekennzeichnet, daß das Melaninpigment (die Melaninpigmente) durch Oxidation wenigstens einer Indolverbindung oder durch Extraktion des Malanins aus melaninhaltigen Substanzen erhalten wurde(n).

3. Mittel nach Anspruch 2, dadurch gekennzeichnet, daß die Indolverbindung ausgewählt ist unter Verbindungen der Formel (I): 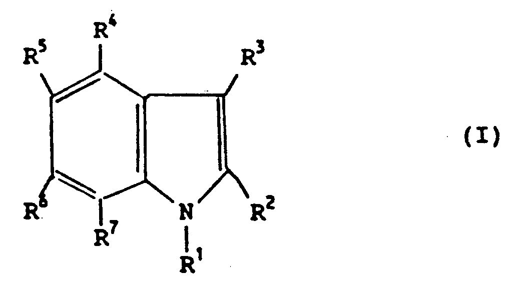 worin:
- R¹ und R³ unabhängig voneinander für Wasserstoff oder C₁-C₄-Alkyl stehen;
- R² für Wasserstoff, C₁-C₄-Alkyl, Carboxyl oder (C₁-C₄-Alkoxy)carbonyl steht;
- R⁴ und R⁷ unabhängig voneinander für Wasserstoff, Hydroxy, C₁-C₄-Alkyl, Amino, C₁-C₄-Alkoxy, (C₂-C₄-Acyl)oxy oder (C₂-C₄-Acyl)amino steht;
- R⁵ für Wasserstoff, Hydroxy, C₁-C₄-Alkoxy, C₁-C₄-Alkyl, Halogen, Amino, (C₂-C₁₄-Acyl)oxy, (C₂-C₄-Acyl)amino oder Trimethylsilyloxy steht;
- R⁶ für Wasserstoff, Hydroxy, C₁-C₄-Alkoxy, Amino, (C₂-C₄-Acyl)oxy, (C₂-C₄-Acyl)amino, Trimethylsilyloxy oder (C₂-C₄-Hydroxyalkyl)amino steht;
- R⁵ und R⁶ zusammen mit den Kohlenstoffatomen, an die sie gebunden sind, auch einen Methylendioxyring, der gegebenenfalls durch eine C₁-C₄-Alkyl- oder C₁-C₄-Alkoxygruppe substituiert ist, oder einen Carbonyldioxyring bilden;
- wenigstens einer der Reste R⁴ bis R⁷ für eine Gruppe OZ oder NHR⁰ steht, wobei höchstens einer der Reste R⁴ bis R⁷ für NHR⁰ steht; und höchstens zwei der Reste R⁴ bis R⁷ für OZ stehen und, wenn Z für Wasserstoff steht, die beiden OH-Gruppen sich in 5- und 6-Position befinden; und wenigstens einer der Reste R⁴ bis R⁷ für Wasserstoff steht und, wenn einer dieser Reste für Wasserstoff steht, einer der Reste R⁴ bis R⁷ dann für NHR⁰ oder OZ steht, wobei die anderen Reste für C₁-C₄-Alkyl stehen;
- der Rest R⁰ der Gruppe NHR⁰ für Wasserstoff, C₂-C₄-Acyl, Hydroxy-C₂-C₄-alkyl steht und der Rest Z der Gruppe OZ für Wasserstoff, C₂-C₁₄-Acyl, C₁-C₄-Alkyl oder Trimethylsilyl steht,
und den Alkalimetall-, Erdalkalimetall-, Ammonium- oder Aminsalzen davon.

4. Mittel nach Anspruch 3, dadurch gekennzeichnet, daß die Indolverbindung ausgewählt ist unter 4-Hydroxyindol, 5-Hydroxyindol, 6-Hydroxyindol, 7-Hydroxyindol, 4-Hydroxy-5-methoxyindol, 4-Hydroxy-5-ethoxyindol, 2-Carboxyl-5-hydroxyindol, 5-Hydroxy-6-methoxyindol, 6-Hydroxy-7-methoxyindol, 5-Methoxy-6-hydroxyindol, 5,6-Dihydroxyindol, N-Methyl-5,6-dihydroxyindol, 2-Methyl-5,6-dihydroxyindol, 3-Methyl-5,6-dihydroxyindol, 2,3-Dimethyl-5,6-dihyroxyindol, 2-Carboxyl-5,6-dihydroxyindol, 4-Hydroxy-5-methylindol, 2-Carboxyl-6-hydroxyindol, 6-Hydroxy-N-methylindol, 2-Ethoxycarbonyl-5,6-dihydroxyindol, 4-Hydroxy-7-methoxy-2,3-dimethylindol, 4-Hydroxy-5-ethoxy-N-methylindol, 6-Hydroxy-5-methoxy-2-methylindol, 6-Hydroxy-5-methoxy-2,3-dimethylindol, 6-Hydroxy-2-ethoxycarbonylindol, 7-Hydroxy-3-methylindol, 5-Hydroxy-6-methoxy-2,3-dimethylindool, 5-Hydroxy-3-methylindol, 5-Acetoxy-6-hydroxyindol, 5-Hydroxy-2-ethoxycarbonylindol, 6-Hydroxy-2-carboryl-5-methylindol, 6-Hydroxy-2-ethoxycarbonyl-5-methoxyindol, 6-N-β-Hydroxyethylaminoindol, 4-Aminoindol, 5-Aminoindol, 6-Aminoindol, 7-Aminoindol, N-Methyl-6β-hydroxyethylaminoindol, 6-Amino-2,3-dimethylindol, 6-Amino-2,3,4,5-tetramethylindol, 6-Amino-2,3,4-trimethylindol, 6-Amino-2,3,5-trimethylindol, 6-Amino-2,3,6-trimethylindol, 5,6-Diacetoxyindol, 5-Methoxy-6-acetoxyindol, 5,6-Dimethoxyindol, 5,6-Dibenzyloxyindol, 5,6-Methylendioxyindol, 5,6-Trimethylsilyloxyindol, Phosphorsäureester von 5,6-Dihydroxyindol und den Additionssalzen dieser Verbindungen.

5. Mittel nach einem der Ansprüche 1 bis 4, dadurch gekennzeichnet, daß das Melaninpigment (oder die Melaninpigmente) mit wenigstens einem teilchenförmigen Füllstoff assoziiert ist (oder sind).

6. Mittel nach Anspruch 5, dadurch gekennzeichnet, daß das Melaninpigment (oder die Melaninpigmente) aus der Oxidation wenigstens einer Indolverbindung der wie in einem der Ansprüche 3 und 4 definierten Formel (I) in Mischung mit dem Füllstoff in einem Milieu, das den erwähnten Füllstoff im wesentlichen nicht löst, oder aus der oxidierenden Polymerisation von Melaninprekursoren auf dem Füllstoff resultiert.

7. Mittel nach einem der Ansprüche 5 und 6, dadurch gekennzeichnet, daß der teilchenförmige Füllstoff ein inerter mineralischer Füllstoff mit einer Korngröße von kleiner als 20 000 nm ist.

8. Mittel nach einem der Ansprüche 5 und 6, dadurch gekennzeichnet, daß der teilchenförmige Füllstoff ein inerter Polymerfüllstoff mit einer Teilchengröße von kleiner als 100 000 nm ist, der ausgewählt ist unter natürlichen oder synthetischen, organischen oder anorganischen Polymeren mit vernetzter, kristalliner oder amorpher Struktur und mit einem Molekulargewicht im Bereich von 5000 bis 5 000 000.

9. Mittel nach Anspruch 8, dadurch gekennzeichnet, daß die Polymeren ausgewählt sind unter Polymeren, die abgeleitet sind von Keratin, Chitin, Cellulose, Polyamiden und Homo- oder Copolymeren mit vernetzter, kristalliner oder amorpher Struktur, die aus der Polymerisation von aliphatischen oder aromatischen, mono- oder polyethylenischen Monomeren resultieren.

10. Mittel nach Anspruch 9, dadurch gekennzeichnet, daß die von Keratin abgeleiteten Polymeren ausgewählt sind unter tierischen oder menschlichen Keratinen; chemisch durch Hydrolyse oder Oxidation modifizierten Keratinen mit einem Molekulargewicht im Bereich von 10 000 bis 250 000 und Seidenfibroin.

11. Mittel nach Anspruch 9, dadurch gekennzeichnet, daß die von Chitin abgeleiteten Polymeren Chitin und/oder dessen desacetyliertes Derivat sind.

12. Mittel nach Anspruch 9, dadurch gekennzeichnet, daß die Cellulosepolymeren mikrokristalline Cellulosen sind.

13. Mittel nach Anspruch 9, dadurch gekennzeichnet, daß die synthetischen Polymeren ausgewählt sind unter Polyethylen, Polypropylen, Polystyrol, Poly(methymethacrylat) und vernetztem Poly(methylmethacrylat).

14. Mittel nach Anspruch 9, dadurch gekennzeichnet, daß das Polymer vernetztes Poly-β-alanin ist.

15. Mittel nach Anspruch 8, dadurch gekennzeichnet, daß die Polymere hohle Mikrokügelchen aus einem Copolymer von Vinylidenchlorid und Acrylnitril oder poröse Mikrokügelchen aus Polyamid 12, Polyamid 6 oder Copolyamid 6/12 sind.

16. Mittel nach Anspruch 8, dadurch gekennzeichnet, daß die Polymere Mikroschwämme sind, die aus vernetzten Styrol/Divinylbenzol-, Methylmethacrylat/Ethylenglycoldimethacrylat- oder Vinylstearat/Divinylbenzol-Polymeren gebildet sind.

17. Mittel nach Anspruch 8, dadurch gekennzeichnet, daß die polymere Siliconpulver sind.

18. Mittel nach einem der Ansprüche 5 und 6, dadurch gekennzeichnet, daß der teilchenförmige Füllstoff ein Füllstoff ist, der gebildet ist durch organische oder mineralische Partikel mit lamellarer Struktur und mit einer Teilchengröße von weniger als 50 000 nm.

19. Mittel nach Anspruch 18, dadurch gekennzeichnet, daß die Partikel mit lamellarer Struktur ausgewählt sind unter L-Lauroyllysin, gegebenenfalls mit Zirkoniumpulver beschichteten keramischen Mikropartikeln, lamellarem Titandioxid, lamellarem Talkum, Bornitrid, lamellarem Glimmer, und Bismutoxichlorid.

20. Mittel nach einem der Ansprüche 18 und 19, dadurch gekennzeichnet, daß die Partikel mit lamellarer Struktur Blättchen sind, bei denen das Verhältnis von größter Abmessung zu Dicke im Bereich von 2 bis 100 liegt.

21. Mittel nach einem der Ansprüche 1 bis 20, dadurch gekennzeichnet, daß das die Lipidphase der Vesikel bildende Lipid (oder die die Lipidphase der Vesikel bildenden Lipide) ausgewählt ist (oder sind) unter nicht-ionischen amphiphilen Lipiden, ionischen amphiphilen Lipiden und Gemischen davon.

22. Mittel nach Anspruch 21, dadurch gekennzeichnet, daß die nicht-ionischen amphiphilen Lipide ausgewählt sind unter
(1) geradkettigen oder verzweigten Polyglycerinethern der Formel: 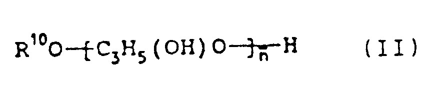 worin:
· -C₃H₅(OH)O für die folgenden Strukturen einzeln oder in Kombination steht:
-CH₂CHOHCH₂O- ; 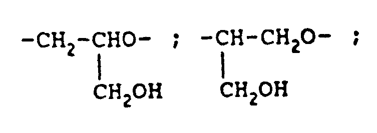
· n̅ einen statistischen Mittelwert von 2 bis 6 bedeutet;
· R¹⁰ bedeutet:
(a) eine geradkettige oder verzweigte aliphatische Kette mit 12 bis 18 Kohlenstoffatomen;
(b) einen Rest R¹¹CO, worin R¹¹ für einen linearen oder verzweigten aliphatischen C₁₁-C₁₇-Rest steht;
(c) einen Rest R¹²⁅OC₂H₃(R¹³)⁆, worin:
· R¹² die für R¹⁰ angegebenen Bedeutungen (a) oder (b) annehmen kann;
· OC₂H₃(R¹³)- die folgenden Strukturen einzeln oder in Kombination darstellt: 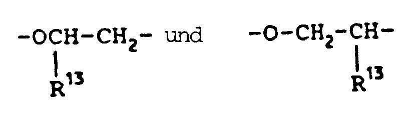 worin R¹³ die für R¹⁰ angegebene Bedeutung (a) besitzt;
(2) polyoxyethylenierten Fettalkoholen;
(3) gegebenenfalls polyoxyethylenierten Polyolestern;
(4) Cerebrosiden; und
(5) oxyethyleniertem Polyglycerinstearat.

23. Mittel nach einem der Ansprüche 21 bis 22, dadurch gekennzeichnet, daß die Lipide mit wenigstens einem stabilisierenden Additiv assoziiert sind, das ausgewählt ist unter Sterinen und anionischen Stabilisierungsmittel.

24. Mittel nach einem der Ansprüche 21 bis 23, dadurch gekennzeichnet, daß die Vesikel einen mittleren Durchmesser im Bereich von 10 bis 1000 nm aufweisen.

25. Mittel nach einem der Ansprüche 1 bis 24, dadurch gekennzeichnet, daß die Lipidphase der Vesikel, die durch das Lipid (oder die Lipide) und das (oder die) Stabilisierungsmittel, mit dem (denen) sie assoziiert sind, gebildet wird, 0,1 bis 16 Gew.-% des gesamten Mittels ausmacht, wobei der Anteil an Stabilisierungsmittel(n) 10 Gew.-% des gesamten Mittels nicht übersteigt.

26. Mittel nach einem der Ansprüche 1 bis 25, dadurch gekennzeichnet, daß die Vesikel in einem Anteil von 0,5 bis 15 Gew.-%, bezogen auf das Gesamtgewicht der Zusammensetzung, vorhanden sind.

27. Mittel nach einem der Ansprüche 1 bis 26, dadurch gekennzeichnet, daß das Melaninpigment (oder die Melaninpigmente), gegebenenfalls auf dem Füllstoff, in einem Anteil von 0,01 bis 5 Gew.-%, bezogen auf das Gesamtgewicht des Mittels, vorhanden sind.

28. Mittel nach einem der Ansprüche 1 bis 27, dadurch gekennzeichnet, daß es in Form einer Lotion, eines Gels, einer Creme und einer Milch vorliegt.

29. Mittel nach einem der Ansprüche 1 bis 28, dadurch gekennzeichnet, daß es wenigstens ein kosmetisch akzeptables Additiv enthält, das ausgewählt ist unter Dihydroxyaceton, Silikonen, Verdickungsmitteln, Mineralölen, Pflanzenölen, Fettsäureestern, Fettalkoholen, Ultraviolettfilter, mineralischen Pigmenten, Konservierungsmittel, Parfüms, alkalisch-machenden Mitteln, sauer-machenden Mitteln, Stabilisierungsmitteln und Farbstoffen.

30. Kosmetische Verwendung des Mittels nach einem der Ansprüche 1 bis 29 zum Schutz der Haut oder der Haare vor Ultraviolettstrahlen oder zur Pigmentierung der Haut oder der Haare.

31. Kosmetische Verwendung des Mittels nach einem der Ansprüche 1 bis 29 zum Schminken oder zur Färbung der Haare.
